# EUROPEAN PATENT APPLICATION

(11) **EP 3 903 799 A1**
(43) Date of publication of application: **03.11.2021**
(21) Application number: 20172098.4
(22) Date of filing: 29.04.2020
(51) Int. Cl.: A61K 36/25, A61K 36/38, A61K 36/63

(54) **METHOD FOR DELIPIDATING PLANT EXTRACTS**

(71) Applicant: Max Zeller Söhne AG, 8590 Romanshorn (CH)
(72) Inventor: BOONEN, Georg, 8590 Romanshorn (CH); KREUTER, Matthias-Heinrich, 8880 Walenstadt (CH); DISCH, Lucia, 8590 Romanshorn (CH)
(74) Representative: Kasche & Partner

(57) **Abstract**

The present invention is directed to a method for preparing plant extracts comprising the steps of (i) preparing a soluble extract from (a) plants or parts thereof and (b) an aqueous, alcoholic or aqueous alcoholic mixed extractant; and removal of insoluble components, (ii) concentrating the extract to a dry substance content at which the contained lipids and lipoids form suspended and/or emulgated aggregates, (iii) separating the suspended and/or emulgated aggregates by microfiltration from the concentrated extract, and (iv) optionally removing the extractant. The invention further pertains to an extract and composition comprising said extract as prepared by this method.

## Description

The present invention is directed to a method for preparing plant extracts comprising the steps of (i) preparing a soluble extract from (a) plants or parts thereof and (b) an aqueous, alcoholic or aqueous alcoholic mixed extractant; and removal of insoluble components, (ii) concentrating the extract to a dry substance content at which the contained lipids and lipoids form suspended and/or emulgated aggregates, (iii) separating the suspended and/or emulgated aggregates by microfiltration from the concentrated extract, and (iv) optionally removing the extractant. The invention further pertains to an extract and composition comprising said extract as prepared by this method.

### Background of the invention

The above-ground parts of plants feature overcoats of lipids and lipoids, in particular waxes for protection against environmental threats, for example, heat, cold, moisture, UV-irradiation, water loss and microbial assaults. These overcoat compositions can vary substantially from plant to plant, depending on climate and the specific part of the plant.

Medicinal plants for modern phytotherapy are often administered as extracts that retain the active composition of the plant and are desired to be equivalent to the plant as such. Extracts typically have the advantage of improved dosing, improved stability, high bioavailability and a more uniform range of active agents. For a most complete extraction of a broad polarity range of different ingredients aqueous alcoholic, e.g. aqueous ethanol mixtures are employed for extraction. Dried plant extracts are regularly stable, should dissolve well or completely in aqueous media, e.g. gastric and intestinal liquid, and feature identical properties from batch to batch. In particular, extracts formulated as tablets and capsules should disintegrate fast and completely to secure the bioavailability of the active ingredients.

However, at normal body temperature plant lipids and lipoids cannot be wetted or dissolved by aqueous media, and enclose or coat hydrophilic extract components which significantly prevents or at least delays the dissolution of active extract components.

Complicating the situation further, the contents of lipids and lipoids in plant raw materials vary naturally and will be introduced into plant extract to varying degrees, thus leading to batch variation and lack of "content conformity", a critical factor for extract-based solid drug forms.

Lipophilic solvents such as supercritical carbon dioxide, petroleum benzine, hexane or chlorinated hydrocarbons are often toxic and/or flammable and have utility in the food industry, e.g. for degreasing flour from oilseeds. However, in phytomedicine, these lipophilic solvents regularly lead to the complete loss of active ingredients. Other methods for separating lipids and lipoids from plant extracts such as precipitation, decanting and centrifugation of lipidic slurries are not satisfactory because the multicomponent extracts regularly do not allow for a clear phase separation and result in loss of active ingredients.

US 6,024,998 discloses a process for removing lipophilic contaminations in beverages and vegetable preparations by (i) mixing said materials with a lipophilic phase, thereby removing and concentrating the lipophilic contaminations into the lipophilic phase nearly quantitatively, (b) separating the lipophilic phase from the beverage or vegetable preparation, e.g. by liquid-liquid, liquid-solid, freeze put or membrane separation, and (c) obtaining the so purified beverage or vegetable preparation. This process is specifically designed and intended for removing chemical toxins such as pesticides, pest control agents, environmental poisons, organic solvents and plant protective agents by lipophilic phase extraction. Because the lipophilic phase does not only dissolve and remove contaminants but also desirable lipophilic ingredients, e.g. active ingredients in plant extracts, the isolation of these ingredients from the lipophilic phase and addition to the hydrophilic phase is proposed, e.g. isolation by water vapor distillation. For vegetable preparations which contain desirable alkaloids the addition of an acid and subsequent alkaloid salt formation for rendering these alkaloids water soluble is noted. Clearly, the authors of this documents are aware of the problems associated with lipophilic phase extraction for removing contaminants in beverage and vegetable products.

In view of the above, it is the objective of the present invention to provide an improved method for removing lipids and lipoids, in particular epicuticular lipids and lipoids, from plant extracts without loss of physiologically active, in particular medically active substances in the extract. It is a further objective to provide an improved method for preparing plant extracts, wherein the resulting plant extracts are improved in solubility in physiological media, e.g. water, gastric and intestinal liquid, are improved in disintegration properties when form-pressed, e.g. into solid forms such as e.g. tablets, and/or have less batch by batch variance.

This objective is solved by a method for preparing plant extracts, optionally for medical use, comprising the steps of:
(i) preparing a soluble extract from (a) plants or parts thereof and (b) an aqueous, alcoholic or aqueous alcoholic mixed extractant; and removal of insoluble components, e.g. sediments;
(ii) concentrating the extract to a dry substance content at which the contained lipids and lipoids form suspended and/or emulgated aggregates;
(iii) separating the suspended and/or emulgated aggregates by microfiltration from the concentrated extract, and
(iv) optionally removing the extractant.

It was experimentally confirmed that the method of the invention significantly, essentially completely removes lipids and lipoids from the so-treated extracts without removing relevant active ingredients. And the extracts feature improved properties with regard to their disintegration properties when pressed, e.g. tableted, and to their release properties and consequently batch to batch consistency due to the absence of lipids and lipoids. This has been demonstrated for a number of pharmaceutically active ingredients in different phytoceutical extracts, where the active ingredients were retained, whereas lipids and lipoids were essentially removed.

The term "lipoid or lipoid compound", as used herein, refers to a fat-like but polar residue(s)-containing lipid, e.g. glykolipids, phospholipids, membrane lipids, steroids, carotinoides, cutin or cutan.

The microfiltration of step (iii) of the present invention can be executed, e.g. by suitable glass, metal, ceramic, synthetic or polymeric membranes. Optionally the microfiltration is executed with a membrane having a pore size of about 0.01 to about 50 µm, optionally with a pore size of about 0.02 to about 10 µm.

The microfiltration can be performed as single or multiple separate microfiltrations, optionally with reduced pore size from one microfiltration step to the next in order to avoid clogging, for example, when starting with a too small pore size. The microfiltration may be performed by means of a crossflow filtration. The technique "cross flow filtration" is common general knowledge. Alternatively, the crossflow filtration may be performed as a double filtration with a larger pore size for the first filtration step to avoid clogging, and a second filtration step with a smaller pore size for essentially quantitative removal of suspended and/or emulgated aggregates of lipids and lipoids.

In one embodiment of the present invention the microfiltration of step (iii) is a crossflow filtration, optionally a two step microfiltration, optionally featuring a first microfiltration with a pore size of about 0.8 to about 10 µm, optionally about 0.5 to about 2 µm or about 0.5 to about 1.5 µm, followed by a second microfiltration with a pore size of about 0.01 to about 0.8 µm, optionally a pore size of about 0.1 to about 0.6 µm or a pore size of about 0.2 µm.

In step (ii) of the method of the invention the extract is concentrated to a dry substance content at which the contained lipids and lipoids form suspended and/or emulgated aggregates.

The preparation of suspended and/or emulgated aggregates of lipids and/or lipoids is common general knowledge, in particular, in the food industry. For example, suspended and/or emulgated aggregates may be prepared by oversaturation of micelles (see Rudolf Voigt, Lehrbuch der pharmazeutischen Technologie: Solubilisierung; Theorie und experimentelle Angaben über Mizellen; p. 491-493, 4. Ed., Verlag Chemie Weinheim-NewYork, 1982) or by an agglomeration process regularly employed in the dairy industry (see Rudolf Heiss, Lebensmitteltechnologie: Biochemische, chemische, mechanische und thermische Verfahren zur Lebensmittelverarbeitung: Butter, Butterungsverfahren, Schaumbutterungsverfahren, p. 13-15, 6. Ed., Springer Verlag Berlin-Heidelberg-New York, 2004)". Formation of suspended and/or emulgated aggregates regularly coincides with the onset of turbidity, which is a directly visible indicator, e.g. the concentrated solution turns opaque.

For example, the complete or essentially complete removal of suspended and/or emulgated lipid/lipoid-aggregates can be observed with the naked eye when comparing the liquid before and after microfiltration(s). Alternatively, the quantitative removal of suspended and/or emulgated lipid/lipoid-aggregates can be verified by a turbity measurement according to ISO 7027 (DIN EN 27027), e.g. results in NTU units (Nephelometric Turbidity Unit).

In the method of the present invention it is these suspended and/or emulgated aggregates that allow for the essentially complete microfiltration and, thus removal of the lipids and lipoidsaggregates without loss of active ingredients in the extract.

It is evident to those skilled in the art that the formation of suspended and/or emulgated aggregates as well as the pore size(s) for their microfiltration(s) will vary with the specific plant material, the extractant and other factors, and that the necessary parameters can be determined by routine optimization and without undue diligence.

It has been shown that the dry substance content upon concentration according to step (ii) can be correlated with the generation of agglomerated micelles. In one embodiment, the dry substance content in step (ii) is about 5 to about 50 wt-%, optionally about 3 to about 30 wt-% or about 6 to about 18 wt-%.

Optionally, the suspended and/or emulgated aggregates formed in step (ii) have a diameter of at least about 100 nm to at least about 1000 µm, optionally of at least about 200 nm to at least about 50 µm or of at least about 500 nm to at least about 10 µm.

In one embodiment the agglomerated micelles formed in step (ii) comprise one or more lipids and/or lipoids, optionally epicuticular lipids and/or lipoids, optionally selected from the group consisting of waxes, optionally C₁₆₋₃₆ fatty acid esters, C₂₄₋₂₈ wax alcohols, C_{28,30,33,36} paraffins, C₁₆₋₃₆ carbohydrates, hydrophobic aliphatic compounds, phytosterines and phytosteroles.

The extractant in step (i) for preparing the initial extract in the method of the present invention is an aqueous, alcoholic or aqueous alcoholic mixed extractant. In one alternative for practicing the present invention the extractant is selected from the group consisting of water, alcohols selected from the group consisting of C₁₋₅alcohols including structural isomers thereof, optionally methanol and ethanol; aqueous alcoholic mixtures of C₁₋₅alcohols including structural isomers thereof, optionally methanol and ethanol, optionally in concentrations 5 to 90, 10 to 80 or 30 to 70 wt-% alcohol content.

The general suitability of the method of the invention for effectively removing lipids and lipoids from plant extracts has been demonstrated for a number of different plant species and plant materials. Therefore, the plants or plant parts for use in the method of the present inventtion may be optionally selected from the group consisting of Hypericum sp., Hedera sp., Olea europaea, Allium sp., optionally A. cepa L., A. sativum L., Arctostaphylos uva-ursi Spreng, Armoracia sp., Artemisia absinthium L, Artemisia annua L, Betula sp., Brassica sp., Camellia sinensis (L.) O. Kuntze, Cassia angustifolia Vahl, Cassia senna L., Cestrum-diurnum; Cucurbita sp.. Echinacea sp., Ginkgo biloba L., Humulus lupulus L., Hypericum perforatum L., Ilex paraguariensis St. Hil., Laurus nobilis L., Marrubium vulgare L., Matricaria recutita L., Melissa officinalis L., Mentha species, Moringa oleifera (Syn.: Guilandina moringa), Orthosiphon stamineus Benth., Passiflora incarnata L., Petasistis hybridus L., Piper sp., Salvia sp., Solanum glaucophyllum, Thymus vulgaris L., Tropaeolum-Arten, and Vitex agnus castus L. In one embodiment, plants or plant parts for use in the method of the present invention are Hypericum sp., Hedera sp. and/or Olea europaea.

The method of the present invention leads to novel extract composition with novel properties due to the essentially quantitative removal of lipids and lipoids. For example, these extracts have improved disintegration properties when pressed, e.g. tableted, and feature improved active ingredient releasing properties. Therefore, the present invention is also directed to those extracts prepared by the method of the present invention as described herein.

In a further aspect, the present invention relates to those compositions, optionally a medical composition, comprising an extract prepared by the method of the present invention as described herein, in particular, for use in the treatment of a disease.

For example, the extracts prepared according to the present invention may be formulated pharmaceutically according to standard procedures, e.g. as recited in standard textbooks such as Gaedcke & Frauke: Phytopharmaka: wissenschaftliche und rechtliche Grundlagen für die Entwicklung, Standardisierung und Zulassung in Deutschland und in Europa; Extrakte, Tinkturen, Drogenzubereitungen, Extraktzubereitungen, Frischpflanzenzubereitungen, p. 3-7; Stuttgart: Wissenschaftliche Verlagsgesellschaft, 2000; and Hänsel, Rudolf & Sticher, Otto: Pharmakognosie, Phytopharmazie; Pflanzliche Arzneizubereitungen, Trockenextrakte als Arzneistoff: Herstellung, Qualitätsprüfung; p. 218-243, 8. Ed., Springer Medizinverlag, Heidelberg, 2007, and they may be administered the same as corresponding plant extracts and their formulations.

In the following the invention will be illustrated by representative examples, none of which are to be interpreted as limiting the scope of the invention beyond the appended claims.

### Example 1

### Process step 1

2505 kg dried and cut (cutting size 80% < 2,5 mm) St John's wort (PhEur) were introduced into 8 parts by weight 50 wt-% ethanol. This mixture was stirred in a flask with stirring, heating and cooling apparatus at a temperature of 50+10°C for 50+15 min. Then it was filtered over a band filter. The obtained liquid extract was concentrated at 100 mbar (max. 200 mbar) and 50°C+15°C in a vacuum evaporator to a dry substance content of 20+10 wt-%t. 3947 kg of turby, thin liquid extract with a dry substance weight of 14.97 wt-% resulted. The extract was left at room temperature for 24 h to settle slurry-like sediments. Subsequently, the fluid was decanted and the sediments discarded. An opaque, emulsion-like liquid with remaining suspended components resulted.

### Process step 2

Of the above material 2000 kg (±10 wt-%) extract were subjected to a crossflow filtration via a polypropylene membrane with a pore size of 1 µm (Seprodyn®) at 20+10°C and 1±1bar. An opaque, emulsion-like liquid resulted.

### Process step 3

The obtained permeate of 1850kg (±10 wt-%) was then microfiltered by crossflow filtration via a polyethylene membrane with a pore size of 0.2 µm (Microdyn®). A clear fluorescing (in transmitted light) liquid resulted.

### Process step 4

The obtained permeate was then concentrated in an evaporator at increased temperature and reduced pressure to a dry substance content of 50 wt-% (±10%). The obtained thick extract was converted into a dry extract by spray band drying.

### Analytical data

273 kg dry extract with a hypericin content (PhEur) of 0.22 wt-% and a flavonoid content (PhEur) of 10.15 wt-% resulted. In order to verify if the two microfiltrations completely or essentially completely preserved the desired ingredients samples were repeatedly taken from the permeates and retentates and analyzed for their content of hypericins and flavonoids.

### Filtration step Seprodyn

In the permeate of the Seprodyn filtration the content of hypericins averaged 0.25 wt-%, and for flavonoids 9.87 wt-% relative to the dry substance. In the retentate of the Seprodyn filtration 0.25 wt-% hypericins and 9.87 wt-% flavonoids were detected.

### Filtration step Microdyn

In the permeate of the Microdyn filtration the content of hypericins averaged 0.25 wt-% and for flavonoids 10.17 wt-% relative to the dry substance. In the retentate of the Microdyn filtration 0.23-0.26 wt-% hypericins and 9.99-10.17 wt-% flavonoids were detected.

These contents demonstrate that the filtrations do not reduce the desired ingredients. Consequently, a 100% or almost 100% yield of the desired ingredients resulted.

### Test for petroleum ether soluble lipoids/lipids

A sample of 10.0 g of the dry extract was transferred to an Erlenmeyer flask with ground-in stopper and magnetic stir bar and intensely stirred with 10 parts petroleum ether for 2 hours at 500 rpm and room temperature. Filtration by means of a cellulose folded filter followed and the obtained petroleum ether phase was evaporated in a tared flask to dryness (300-50 mbar, 40°C). The tared flask was then weighed on an analytical scale. The flask did not show any added weight and the sample did not contain any petroleum ether soluble lipoids/lipids.

### Example 2 (comparison)

### Process step 1

9362 kg dried and cut (cutting size 80% < 2,5mm) St John's wort (PhEur) were introduced into 8 parts by weight 50 wt-% ethanol. This mixture was stirred in a flask with stirring, heating and cooling apparatus at 50+10°C for 50+15 min. Then it was filtered over a band filter. The obtained liquid extract was concentrated at 100 mbar (max. 200 mbar) and 50+15°C in a vacuum evaporator to a dry substance content of 20+10 wt-%. 7798 kg of turby, thin liquid extract with a dry substance content of 25.0 wt-% resulted. The extract was left at room temperature for 24 h to settle slurry-like sediments. Subsequently, the fluid was decanted and sediments discarded. An opaque, emulsion-like liquid with suspended components resulted.

### Process step 2

Then the extract was concentrated in an evaporator at elevated temperature and reduced pressure to a dry substance content of 50+10 wt-%. The obtained thick extract was transformed into a dry extract by spray band drying.

Analytical data:
1873 kg dry extract with a hypericin content (PhEur) of 0.12 wt-% and a flavonoid content (PhEur) of 8.4 wt-% resulted.

### Test for petroleum ether soluble lipoids/lipids

A sample of 10.0 g of the dry extract was transferred to an Erlenmeyer flask with ground-in stopper and magentic stir bar and intensly stirred with 10 parts petroleum ether for 2 hours at 500 rpm and room temperature. Filtration by means of a cellulose folded filter followed and the obtained petroleum ether phase was evaporated in a tared flask to dryness (300-50 mbar, 40°C). The tared flask was then weighed on an analytical scale. The flask showed an added weight of 0.43 g and the sample contained 4.3 wt-% petroleum ether soluble lipoids/lipids. The example 2 contained 4.3 fold the amount of lipoids/lipids compared to example 1.

### Example 3

273.9 kg dry extract obtained according to example 1 were mixed with 1.5 kg highly dispersed silicon dioxide (Aerosil®), 22.2 kg polyoxyethylenglycol (PEG 6000) and 2.4 kg magnesium stearate resulting in 300 kg of a pelleting mixture. 300 g of this mixture were pressed to tablet cores with a weight of 539.9 mg. These tablet cores were tested for hardness, disintegration and release time (based on the release of total hypericins in a dissolution test model: required: >75 % release within 60 min). The tablets had a hardness of 76 Newton, disintegrated completely in 9.49 min and released within 20 min over 75 % of the total hypericin active agents.

### Example 4 (comparison)

273.9 kg dry extract obtained according to example 2 (comparison) were mixed with 1.5 kg highly dispersed silicion dioxide (Aerosil®), 22.2 kg polyoxyethylenglycol (PEG 6000) and 2.4 kg magnesium stearate resulting in 300 kg of a pelleting mixture. 300 g of this mixture were pressed to tablet cores with a weight of 543,3 mg. These tablet cores were tested for hardness, disintegration and release time (based on the release of total hypericins in a dissolution test model: required: >75 % release within 60 min). The tablets had a hardness of 46 Newton, disintegrated completely in 11.32 min and released 34 % of the total hypericin active agents within 60 min.

### Example 5

### Process step 1

2 kg liquid extract of St John's wort with a dry substance content of 15 wt-% obtained according to example 2 (comparison, process step 1) were diluted with drinking water (aqua fontana, PhHelv) to a dry substance content of 10 wt-%. An opaque, emulsion-like liquid with suspended components resulted.

### Process step 2

Of the above material 3 kg (±10 wt-%) liquid extract were subjected to a crossflow filtration via a polypropylene membrane with a pore size of 1 µm (Seprodyn®) at 20+10°C and 1+ 1 bar. An opaque, emulsion-like liquid resulted.

### Process step 3

The obtained permeate was then microfiltered by crossflow filtration via a polyethylene membrane with a pore size of 0.2 µm (Microdyn®). A clear fluorescing (in transmitted light) liquid resulted.

### Analytical data

The employed liquid extract and both permeates were analyzed for their contents of total hypericins and flavonoids. The liquid extract contained 0.227 wt-% total hypericins and 7.87 wt-% flavonoids relative to the dry substance content. The Seprodyn permeate comprised 0.23 wt-% total hypericins and 7.86 wt-% total flavonoids, and the Microdyn permeate comprised 0.22 wt-% total hypericins and 8.00 wt-% flavonoids relative to the dry substance content. Consequently, a 100 % or almost 100% yield of the desired ingredients resulted.

### Example 6

### Process step 1

1500 g dried and cut (cutting size 80% < 5 mm) olive leaves (PhEur) were introduced into 10 parts by weight 70 wt-% ethanol. This mixture was stirred in a flask with stirring, heating and cooling apparatus at 50+10°C for 50+15 min. Then it was filtered over a layer filter. The obtained liquid extract was concentrated under reduced pressure (300-100 mbar) and 50+15°C in a vacuum evaporator to a dry substance content of 25 (20-35) wt-%. 2128 g of turby, thin liquid extract with a dry substance weight of 20.4 wt-% resulted. An opaque, emulsion-like liquid with suspended components resulted.

### Process step 2

This liquid emulsion was divided in two portions of 1064g. For example 6 the liquid was diluted with the equal amount of tap water (aqua fontana) to a dry substance content of 10+5wt-% and the resulting turby, thin liquid extract was subjected to a crossflow filtration via a polypropylene membrane with a pore size of 1 µm (Seprodyn®) at 20+10°C and 1+1 bar. The death volume of the equipment was roughly 500ml. An opaque, emulsion-like liquid of 1571g was obtained.

### Process step 3

The obtained permeate of 1571 g (±10 wt-%) was then microfiltered by crossflow filtration via a polyethylene membrane with a pore size of 0.2 µm (Microdyn®) at 20+10 °C and 1±1bar. The death volume of the equipment was roughly 500ml. A clear brown shining (in transmitted light) liquid resulted.

### Process step 4

The obtained permeate was mixed with 20 wt-% maltodextrin (PhEur) and then concentrated in an evaporator at increased temperature and reduced pressure to dryness and the obtainned dry extract was milled.

### Analytical Data

The obtained beige-brown powder extract of 111.82 g dry extract had an oleuropein content (PhEur) of 19.31 wt-%. In order to verify if the two microfiltrations completely or essentially completely preserved the desired ingredient dry extract samples were taken and the content compared with the comparison sample described in example 7.

These contents demonstrate that the filtrations do not reduce the desired ingredient. Consequently, a 100 % or almost 100% yield of the desired ingredient resulted.

### Test for petroleum ether soluble lipoids/lipids

A sample of 3.0 g of the dry extract was transferred to an Erlenmeyer flask with ground-in stopper and magnetic stir bar and intensely stirred with 100ml petroleum ether for 30 min at room temperature. Filtration by means of a cellulose folded filter followed and the obtained petroleum ether phase was evaporated in a tared flask to dryness (900-50 mbar, 40°C). The tared flask was then weighed on an analytical scale. The flask did show an added weight of 0.006g, corresponding to a minimal content of petroleum ether soluble lipoids/lipids of 0.2% wt.

### Example 7 (comparison)

### Process step 1

1064 g of the opaque, emulsion-like liquid with suspended components from the end of process step 1 of example 6 were taken for comparison example 7, to allow a direct comparison.

### Process step 2

The obtained liquid extract was mixed with 20 wt-% maltodextrin (PhEur) relative to the dry substance content and then concentrated in an evaporator at increased temperature and reduced pressure to dryness and the obtained dry extract was milled.

### Analytical Data

208.6 g of a greenish-brown dry extract had an oleuropein content (PhEur) of 17.52 wt-%.

### Test for petroleum ether soluble lipoids/lipids

A sample of 3.0 g of the dry extract was transferred to an Erlenmeyer flask with ground-in stopper and magnetic stir bar and intensely stirred with 100ml petroleum ether for 30 min at room temperature. Filtration by means of a cellulose folded filter followed and the obtained petroleum ether phase was evaporated in a tared flask to dryness (900-50 mbar, 40°C). The tared flask was then weighed on an analytical scale. The flask showed a weight increase of 0.043 g, corresponding to a content of petroleum ether soluble lipoids/lipids of 1.43 wt-% or a 7-fold higher content compared to example 6.

### Example 8

### Process step 1

1700 g dried and cut (cutting size 80% < 2 mm) ivy leaves (PhEur) were introduced to 10 parts by weight 40 wt-% ethanol. This mixture was stirred in a flask with stirring, heating and cooling apparatus at 50+10°C for 50+15 min. Then it was filtered over a layer filter. The obtained liquid extract was concentrated under reduced pressure (300-100 mbar) and 50+15 °C in a vacuum evaporator to a dry substance content of 10 (5-15) wt-%.

4970 g of turby, thin liquid extract with a dry substance weight of 6.85 wt-% resulted. An opaque, emulsion-like liquid with suspended components resulted.

### Process step 2

This liquid emulsion was divided into two equal portions (1 portion for comparison example 9), and one portion of the thin liquid extract was subjected to a crossflow filtration via a polypropylene membrane with a pore size of 1 µm (Seprodyn®) at 20+10°C and 1+1 bar. An opaque, emulsion-like liquid resulted.

### Process step 3

The obtained permeate of 1600 g (± wt-%) was then microfiltered by crossflow filtration via a polyethylene membrane with a pore size of 0.2 µm (Microdyn®) at 20+10 °C and 1+1 bar. A clear brown shining (intransmitted light) liquid resulted.

### Process step 4

The obtained brilliant clear permeate was mixed with 20 wt-% maltodextrin (PhEur) relative to the dry substance content and then spray dried. Subsequently the obtained dry extract was milled.

### Analytical Data 57.8 g dry extract had a hederacoside C content (PhEur) of 12.29 wt-%.

### Test for petroleum ether soluble lipoids/lipids

A sample of 3.0 g of the dry extract was transferred to an Erlenmeyer flask with ground-in stopper and magnetic stir bar and intensely stirred with 100ml petroleum ether for 30 min at 500 rpm and room temperature. Filtration by means of a cellulose folded filter followed and the obtained petroleum ether phase was evaporated in a tared flask to dryness (900-50 mbar, 40°C). The tared flask was then weighed on an analytical scale. The flask did show an added weight of 0.006g corresponding to a minimal content of 0.2 wt% petroleum ether soluble lipoids/lipids.

### Example 9 (comparison)

### Process step 1

2485 g of the opaque, emulsion-like liquid with suspended components from end of process step 1 of example 8 were taken for the comparison example 9, to allow a direct comparison.

### Process step 2

The obtained cloudy liquid extract was mixed with 20 wt-% maltodextrin (PhEur) relative to the dry substance content and spray dried. The obtained dry extract was milled.

### Analytical Data

104.3 g dry extract with a hederacoside C content (PhEur) of 13.77 wt-% resulted.

Test for petroleum ether soluble lipoids/lipids

A sample of 3.0 g of the dry extract was transferred to an Erlenmeyer flask with ground-in stopper and magnetic stir bar and intensely stirred with 100 ml petroleum ether for 30 min at 500 rpm and room temperature. Filtration by means of a cellulose folded filter followed and the obtained petroleum ether phase was evaporated in a tared flask to dryness (900-50 mbar, 40°C). The tared flask was then weighed on an analytical scale. The flask had an added weight of 0.012 g and did contain 0.4 wt-% petroleum ether soluble lipoids/lipids calculated on dry extract. This is a 2-fold content compared to example 8.

### Example 10

### Process step 1

2000 g dried and cut (cutting size 80% < 2,5 mm) St. Johns wort (PhEur) were introduced to 10 parts by weight 50 wt-% ethanol. This mixture was stirred in a flask with stirring, heating and cooling apparatus at 50+10°C for 40+15 min. Then it was filtered over a layer filter. The obtained liquid extract was concentrated under reduced pressure (300-100 mbar) and 50+15 °C in a vacuum evaporator to a dry substance content of 13.39 (5-15) wt-%.

2560 g of turby, thin liquid extract with a dry substance weight of 6.85 wt-% resulted The extract was left at room temperature for 24 h to settle slurry-like sediments. Subsequently the fluid was decanted and the sediments discarded. An opaque, emulsion-like liquid with suspended components resulted.

### Process step 2

This liquid emulsion was divided into two equal portions (1 portion for comparison example 11) and one portion of the thin liquid extract was subjected to a crossflow filtration via a polypropylene membrane with a pore size of 1 µm (Seprodyn®) at 20+10°C and 1±1 bar. An opaque, emulsion-like liquid resulted.

### Process step 3

The obtained permeate was then microfiltered by crossflow filtration via a polyethylene membrane with a pore size of 0.2 µm (Microdyn®) at 20+10 °C and 1±1 bar. A clear brown shining (in transmitted light) liquid resulted.

### Process step 4

The obtained brilliant clear permeate was mixed with 20 wt-% maltodextrin (PhEur) relative to the dry substance content and then vacuum oven dried. Subsequently the obtained dry extract was milled.

### Analytical Data

The resulting dry extract had a Hypericin content (PhEur) of 0.196 wt-% and a flavonoid content (PhEur) of 7.49 wt%.

### Test for petroleum ether soluble lipoids/lipids

A sample of 3.0 g of the dry extract was transferred to an Erlenmeyer flask with ground-in stopper and magnetic stir bar and intensely stirred with 100ml petroleum ether for 30 min at 500 rpm and room temperature. Filtration by means of a cellulose folded filter followed and the obtained petroleum ether phase was evaporated in a tared flask to dryness (900-50 mbar, 40°C). The tared flask was then weighed on an analytical scale. The flask did show an added weight of 0.003g corresponding to a minimal content of 0.1 wt% petroleum ether soluble lipoids/lipids.

### Example 11 (comparison)

### Process step 1

The second portion of the opaque, emulsion-like liquid with suspended components from end of process step 1 of example 10 were taken for the comparison example 11, to allow a direct comparison.

### Process step 2

The obtained liquid extract was mixed with 20 wt-% maltodextrin (PhEur) relative to the dry substance content and vacuum oven dried. The resulting dry extract was milled.

### Analytical Data

A dry extract with a Hypericin content (PhEur) of 0.197 wt % and a Flavonoid content (PhEur) of 7.366 wt % resulted.

### Test for petroleum ether soluble lipoids/lipids

A sample of 3.0 g of the dry extract was transferred to an Erlenmeyer flask with ground-in stopper and magnetic stir bar and intensely stirred with 100 ml petroleum ether for 30 min at 500 rpm and room temperature. Filtration by means of a cellulose folded filter followed and the obtained petroleum ether phase was evaporated in a tared flask to dryness (900-50 mbar, 40°C). The tared flask was then weighed on an analytical scale. The flask had an added weight of 0.027 g and did contain 0.9 wt-% petroleum ether soluble lipoids/lipids calculated on dry extract. This is a 9-fold higher content compared to example 10.

### Summary of results

The above presented examples demonstrate that the method of the present invention allows for the quantitative or almost quantitative separation of suspended or emulsified aggregates of lipids and/or lipoids from aqueous, alcoholic or aqueous alcoholic plant extracts. The separation of those lipids and/or lipoids is specific and does not lead to a significant decrease of extracted components. The effective separation of the undesired lipids or lipoids leads to signifycantly improved release kinetics of the final drug form, for example, because lipids or lipoids cause auto-retardation.

## Claims

1. A method for preparing plant extracts, optionally for medical use, comprising the steps of:
(i) preparing a soluble extract from (a) plants or parts thereof and (b) an aqueous, alcoholic or aqueous alcoholic mixed extractant; and removal of insoluble components;
(ii) concentrating the extract to a dry substance content at which the contained lipids and lipoids form suspended and/or emulgated aggregates;
(iii) separating the suspended and/or emulgated aggregates by microfiltration from the concentrated extract, and
(iv) optionally removing the extractant.

2. The method according to claim 1, wherein the membrane for the microfiltration of step (iii) has a pore size of about 0.01 to about 50 µm, optionally a pore size of about 0.02 to about 10 µm.

3. The method according to claim 2, wherein the microfiltration of step (iii) is a crossflow filtration, optionally a two step microfiltration, optionally a first microfiltration with a pore size of about 0.8 to about 10 µm, optionally about 0.5 to about 2 µm or about 0.5 to about 1.5 µm, followed by a second microfiltration with a pore size of about 0.01 to about 0.8 µm, optionally a pore size of about 0.1 to about 0.6 µm or a pore size of about 0.2 µm.

4. The method according to one of claims 1 to 3, wherein the dry substance content in step (ii) is about 5 to about 50 wt-%, optionally about 3 to about 30 wt-% or about 6 to about 18 wt-%.

5. The method according to any one of claims 1 to 4, wherein the suspended and/or emulgated aggregates formed in step (ii) have a diameter of at least about 100 nm to at least about 1000 µm, optionally of at least about 200 nm to at least about 50 µm or of at least about 500 nm to at least about 10 µm.

6. The method according to any one of claims 1 to 5, wherein the suspended and/or emulgated aggregates of step (ii) comprise one or more lipids and/or lipoids, optionally epicuticular lipids and/or lipoids, optionally selected from the group consisting of waxes, optionally C₁₆₋₃₆ fatty acid esters, C₂₄₋₂₈ wax alcohols, C_{28,30,33,36} paraffins, C₁₆₋₃₆ carbohydrates, hydrophobic aliphatic compounds, phytosterines and phytosteroles.

7. The method according to any one of claims 1 to 6, wherein the extractant in step (i) is selected from the group consisting of water, alcohols selected from the group consisting of C₁₋₅alcohols including structural isomers thereof, optionally methanol and ethanol; aqueous alcoholic mixtures of C₁₋₅alcohols including structural isomers thereof, optionally methanol and ethanol, optionally in concentrations 5 to 90, 10 to 80 or 30 to 70 wt-% alcohol content.

8. The method according to any one of claims 1 to 7, wherein the plants or plant parts are selected from the group consisting of Hypericum sp., Hedera sp., Olea europaea, Allium sp., optionally A. cepa L., A. sativum L., Arctostaphylos uva-ursi Spreng, Armoracia sp., Artemisia absinthium L, Artemisia annua L, Betula sp., Brassica sp., Camellia sinensis (L.) O. Kuntze, Cassia angustifolia Vahl, Cassia senna L., Cestrum-diurnum; Cucurbita sp.. Echinacea sp., Ginkgo biloba L., Humulus lupulus L., Hypericum perforatum L., Ilex paraguariensis St. Hil., Laurus nobilis L., Marrubium vulgare L., Matricaria recutita L., Melissa officinalis L., Mentha species, Moringa oleifera (Syn.: Guilandina moringa), Orthosiphon stamineus Benth., Passiflora incarnata L., Petasistis hybridus L., Piper sp., Salvia sp., Solanum glaucophyllum, Thymus vulgaris L., Tropaeolum sp, and Vitex agnus castus L.

9. An extract prepared by a method according to any one of claims 1 to 8.

10. A composition, optionally a medical composition, comprising an extract according to claim 9.

11. A composition according to claim 10 for use in the treatment of a disease.
